**Europäisches Patentamt**

(19) **European Patent Office**

**Office européen des brevets**

(11) Numéro de publication: **0 039 283**
**B1**

(12) # FASCICULE DE BREVET EUROPEEN

(45) Date de publication du fascicule du brevet:
**08.06.83**

(21) Numéro de dépôt: **81400640.9**

(22) Date de dépôt: **23.04.81**

(51) Int. Cl.³: **C 12 P 1/00,** C 12 N 1/00,
C 12 N 9/34, C 12 N 9/28,
C 12 P 37/00

---

(54) **Procédé de culture microbiologique.**

---

(30) Priorité: **24.04.80 FR 8009287**

(43) Date de publication de la demande:
**04.11.81 Bulletin 81/44**

(45) Mention de la délivrance du brevet:
**08.06.83 Bulletin 83/23**

(84) Etats contractants désignés:
**AT DE GB IT NL SE**

(56) Documents cités:
**FR-A-2 189 511**
**FR-A-2 235 195**
**FR-A-2 352 055**

**CHEMICAL ABSTRACTS, vol. 49, no. 15, 10 août 1955,**
**colonne 10430b, Columbus, Ohio, US. A.E. SMIRNOVA:**
**"Yeast autolysate as a source of nitrogen and vitamin**
**for microorganisms"**

(73) Titulaire: **Roquette Frères, F-62136 Lestrem (FR)**

(72) Inventeur: **Devos, Francis, Route de Merville, Morbecque**
**F-59660 Merville (FR)**
Inventeur: **Huchette, Michel, 63, rue du Maréchal Joffre,**
**F-59660 Merville (FR)**

(74) Mandataire: **Koch, Gustave et al, Cabinet**
**PLASSERAUD 84, rue d'Amsterdam, F-75009 Paris (FR)**

---

## Procédé de culture microbiologique

L'invention a pour objet un procédé microbiologique du genre de ceux qui comportent une étape de filtration permettant de séparer, d'une part, notamment les corps cellulaires du microorganisme mis en œuvre et diverses particules et substances, en particulier colloïdales, en suspension et, d'autre part, la phase liquide comportant les principes actifs résultant du procédé microbiologique.

Parmi les procédés microbiologiques en question, on peut citer ceux de fermentation pour la préparation d'enzymes, de vitamines, d'antibiotiques, d'acides organiques ou d'acides aminés.

Jusqu'à ce jour, on a eu généralement recours, pour réaliser l'étape de filtration, à des agents de filtration de nature minérale ou accessoirement cellulosique, les agents minéraux étant choisis parmi les terres de diatomées, les kieselguhrs, les perlites et l'amiante, ces matériaux ayant souvent subi une étape de calcination et de tamisage avant leur utilisation comme agents de filtration.

Cependant, il a parfois été fait appel à des agents de nature amylacée, comme la fécule de pomme de terre, notamment pour la filtration des levures. Mais l'utilisation de produits amylacés a été et demeure peu répandue et reste de toute façon très spécifique.

Les agents de filtration couramment utilisés comme les kieselguhrs ou les terres de diatomées présentent pourtant un inconvénient majeur qui réside dans le caractère éminemment polluant du gâteau de filtration, c'est-à-dire du résidu constitué par l'agent de filtration et les corps microbiens et colloïdaux retenus.

En effet, ce résidu de filtration impose de sérieuses contraintes dues au caractère polluant de sa partie organique. Ces déchets sont actuellement traités de différentes manières. La pratique la plus courante consiste à transporter ces résidus vers des décharges. Une autre solution consiste à les incinérer ou à les sécher, mais se posent alors, en plus des problèmes d'énergie, de graves poblèmes de corrosion dans les séchoirs et incinérateurs, problèmes dûs au fort pourcentage de charges minérales abrasives présentes dans ces résidus.

Aucune de ces solutions n'apparaît donc satisfaisante; par ailleurs, le problème de la revalorisation de ces résidus inhérents à la grande majorité des procédés microbiologiques reste posé.

Une solution plus attrayante consisterait à généraliser la filtration des moûts de culture à l'aide d'agents de nature amylacée et à revaloriser le gâteau de filtration en l'utilisant pour l'alimentation animale. Mais cette solution implique un séchage des gâteaux de filtration et entraîne d'autre part des complications pour les sociétés spécialisées dans les industries de fermentation qui ne disposent que rarement de débouchés dans l'alimentation du bétail.

Aucune solution connue n'est donc, à l'heure actuelle, satisfaisante sur tous les plans et ne permet de remédier aux inconvénients précités, notamment du point de vue de la pollution.

L'invention a pour but, surtout, de proposer une solution particulièrement élégante au problème de l'élimination des gâteaux de filtration, tout en en permettant une revalorisation simple et avantageuse.

La Société demanderesse a, en effet, eu le mérite de trouver que les gâteaux de filtration qui sont à base d'un agent de filtration constitué par un produit amylacé, étaient propres à former, notamment après autolyse, hydrolyse ou autre traitement de solubilisation ou de dégradation, un adjuvant nutritif intéressant pour les milieux de culture utilisés dans les procédés microbiologiques du genre en question. Ces gâteaux de filtration peuvent donc être notamment recyclés dans le milieu de culture principal, sur le lieu même de production. Cette manière de faire permet de trouver sur place une revalorisation intéressante de ces polluants et d'économiser une partie non négligeable des matières nutritives qui constituent le milieu de culture principal, la réutilisation des autolysats ou des hydrolysats du microorganisme ayant en outre bien souvent un effet bénéfique sur la culture de ce même microorganisme.

Le procédé microbiologique perfectionné conforme à l'invention est donc caractérisé par le fait que, consécutivement à la filtration du moût sur un agent de filtration de nature amylacée, le gâteau de filtration est recyclé dans le milieu de culture principal, constituant ainsi une partie des matières nutritives nécessaires à ce dernier, de préférence après avoir été soumis à un traitement de dégradation, notamment une autolyse, au cours duquel les éléments constitutifs des cellules sont partiellement solubilisés.

L'invention pourra être bien comprise à l'aide du complément de description qui suit ainsi que des exemples relatifs à des modes de réalisation avantageux.

Supposant, par conséquent, que dans le cas d'un procédé microbiologique, la filtration est réalisée en ayant recours, à titre d'agent de filtration, à un amylacé, notamment à un ou plusieurs amidons natifs, de préférence de tubercules, on recycle le gâteau de filtration vers le milieu de culture principal, de préférence après l'avoir soumis à un traitement de dégradation, notamment d'autolyse.

Le gâteau de filtration constitue ainsi une partie des matières nutritives nécessaires à la formation dudit milieu de culture principal.

Le traitement de dégradation, prévu de préférence, permet de rendre plus fermentescibles, au sein du milieu principal, les constituants du gâteau de filtration dont l'humidité est en général à environ 80% ou est amenée à cette valeur pour l'incubation, ce gâteau contenant normalement de 25 à 50% en poids de matière amylacée sur la matière sèche.

Pour augmenter la fermentescibilité du gâteau, on peut le traiter à divers pH, diverses températures et ajouter tous types d'enzymes capables d'hydrolyser tout ou partie de ses constituants.

Pour le traitement de dégradation, le gâteau est recueilli dans des cuves, soit tel quel, soit délayé dans de l'eau; amené à un pH compris entre 4,0 et 8,5 et à une température de 20 à 60°C, il subit alors, l'autolyse des corps microbiens qui peut éventuellement être facilitée par addition d'enzymes supplémentaires, notamment des amylases ou des protéases. Les corps microbiens possèdent cependant généralement une activité enzymatique suffisante pour provoquer directement, dans ces conditions, leur propre autolyse.

Les enzymes supplémentaires éventuellement ajoutées seront donc essentiellement des amylases, α-amylase et amyloglucosidase notamment, capables d'hydrolyser le constituant amylacé du gâteau de filtration.

Ces amylases agissent surtout en fin d'autolyse, par montée de la température au-delà de 60°C, température à laquelle débute la gélification d'agents amylacés comme les fécules de pomme de terre ou de manioc.

Lorsque le microorganisme cultivé est lui-même équipé d'amylases, la fécule native ou liquéfiée à l'α-amylase conviendra.

Si le microorganisme exige, pour sa production principale, du dextrose pur, on hydrolyse l'agent amylacé à l'amyloglucosidase. La quantité et les conditions d'action de l'enzyme seront alors fixées, de manière connue en soi, de façon à ce que l'hydrolyse de l'agent amylacé soit pratiquement complète.

Le lysat final est généralement obtenu après 8 à 24 heures d'incubation. Il est alors recyclé dans le milieu de culture principal.

Ce lysat contient une partie des hydrates de carbone provenant de l'agent de filtration, des amino-acides ou peptides, des vitamines du groupe B, des matières minérales, des acides nucléiques, c'est-à-dire les constituants biologiques les plus adaptés à la croissance des microorganismes cultivés.

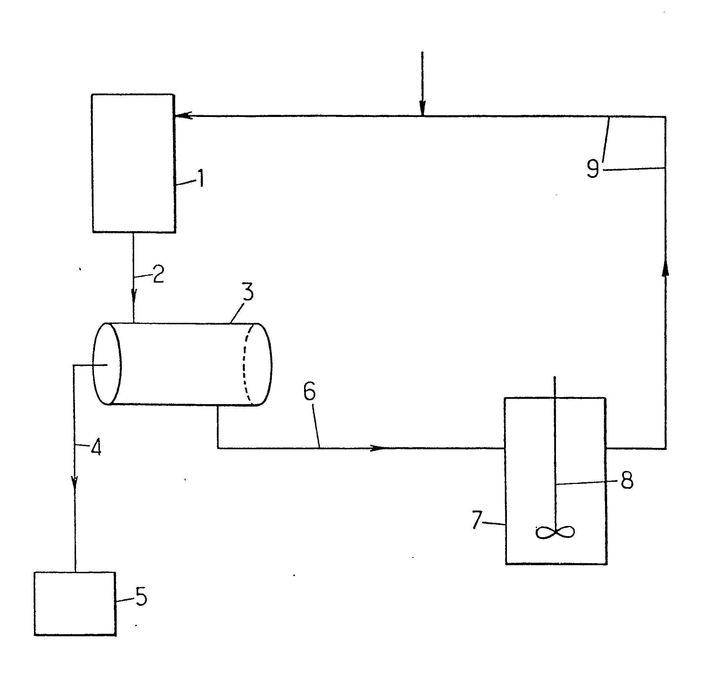

Le procédé conforme à l'invention peut être mis en œuvre en ayant recours à une installation du genre de celle schématiquement montrée à la figure unique.

Cette installation comporte une enceinte 1 dans laquelle se déroule le procédé microbiologique prorement dit.

L'enceinte 1 est reliée par une canalisation 2 à un filtre 3, par exemple du type rotatif («Precoat») comme montré.

A la sortie du filtre 3, on recueille:

– d'une part, par une canalisation 4, le filtrat comportant les principes actifs résultant du procédé et les solubles, ce filtrat pouvant être conduit vers une étape de purification supplémentaire, par exemple une installation d'ultrafiltration 5,
– d'autre part, le gâteau de filtration qui est amené, par exemple par une bande transporteuse 6, à l'une des cuves 7 d'une batterie de cuves d'hydrolyse équipées de moyens d'agitation 8 et à l'intérieur de laquelle il est délayé avec la quantité nécessaire d'eau s'il y a lieu.

Après le temps d'incubation nécessaire, aux conditions indiquées plus haut, le lysat est recyclé vers la cuve 1 grâce à une canalisation 9.

Exemple 1

Production d'amyloglucosidase avec, comme agent de filtration, de la fécule de pomme de terre.

On utilise une souche d'Aspergillus awamori obtenue après de nombreuses mutations aux rayons ultra-violets et à l'aide de divers agents mutagènes.

Le milieu de culture est constitué de 10% de farine de maïs, de 2% de fécule de pomme de terre et de 4% de C.S.L. à 50% de matières sèches (Corn-Steep-Liquor ou «eau de trempage du maïs»).

Dans un fermenteur de laboratoire, on prépare le milieu de culture en mettant en suspension, dans 12 litres d'eau, 2 kg de farine de maïs et 0,4 kg de fécule de pomme de terre. On ajoute 90.000 Unités internationales d'α-amylase et on procède à la liquéfaction de l'amidon de maïs et de la fécule de pomme de terre en maintenant le milieu à une température de 90°C durant 15 minutes.

Après liquéfaction, on ajoute 800 g de C.S.L. dilué dans la quantité d'eau nécessaire pour ajuster le milieu à 20 litres.

Après stérilisation à 120°C pendant 30 minutes environ, le milieu est ensemencé par 300 ml d'une culture de 24 heures d'Aspergillus awamori (milieu farine de maïs à 5% et 2% de C.S.L.).

Après 3 jours de culture sous forte agitation et aération, le taux de substances réductrices libres est tombé à moins de 2 g/litre d'équivalent glucose et l'activité enzymatique mesurée par la méthode standardisée G.A.U. (Glucoamylase, Unit méthode de MILES LABORATORIES) est de 25 unités/ml.

Sur un filtre Büchner de 30 cm de diamètre garni de 500 g de fécule de pomme de terre, on filtre le moût par portions de 1 litre.

La filtration est très facile. Après un lavage à l'aide de 100 cm³ d'eau, on détache aisément la «feuille» (obtenue par un feutrage dû au champignon filamenteux) du gâteau de fécule, ce qui laisse une surface de filtration libre de tout résidu et donc prête à la filtration d'une nouvelle portion d'un litre de moût de culture.

Pour chaque filtration d'un litre de moût de culture, on récupère une «feuille» ou gâteau de filtration d'un poids de 107 g en moyenne.

Au total, on obtient 2,2 kg de gâteau humide à 80% d'eau et dont la composition est la suivante:

1,76  kg d'eau
0,154 kg de fécule (190 g de fécule commerciale)
0,286 kg de cellules.

Ce gâteau est incubé tel quel à une températurere de 50°C et à un pH de 3,8 pendant 12 heures.

La concentration en matières solubles après autolyse est supérieure à 100 g/litre.

Après l'avoir amené à une température de 90°C, on ajoute à cet autolysat cellulaire 400 g de C.S.L. (soit 2% du milieu de culture final) et on ajoute l'ensemble à une nouvelle charge contenant 2 kg de farine de maïs et 0,2 kg de fécule de pomme de terre, préparée dans 12 litres d'eau et liquéfiée comme précédemment (l'autolysat vient donc en lieu et place d'une partie du Corn-Steep et d'une partie de la fécule de pomme de terre présents dans le milieu de culture initial).

Enfin, on ajuste le mélange ainsi constitué à un volume de 20 litres par apport de la quantité d'eau nécessaire. Le processus décrit plus haut est alors de nouveau suivi.

Après 4 jours de fermentation, l'activité enzymatique est de 24 unités G.A.U./ml; la filtrabilité est toujours aussi bonne.

Ce processus a été reproduit quatre fois consécutives. Les activités enzymatiques obtenues ont été de:

24 – 26 – 23 – 25 unités/ml.

Exemple 2
Culture d'un Streptomyces griseus avec filtration sur fécule de pomme de terre et recyclage.

Le Streptomyces a été cultivé sur un milieu contenant:

- malto-dextrine* 1%
- glucose　　　　 5%
- protéine de soja 1%
- C.S.L.　　　　　 2%.

*malto-dextrine: amidon liquéfié à l'α-amylase jusqu'à un dextrose-équivalent de 25.

Après stérilisation et ensemencement par une souche de Streptomyces griseus, la culture a été agitée et aérée pendant 72 heures.

L'activité biologique du moût de culture a atteint alors sa valeur maximum, cette activité coïncidant avec la teneur minimum en glucose dans le moût.

Le moût de culture a été filtré sur un filtre rotatif garni à la fécule de pomme de terre.

Le débit de filtration mesuré a été de 350 litres/m² /heure, ce qui est un débit comparable à celui obtenu sur terre de diatomées.

Le gâteau de filtration lavé titrait 83% d'humidité et présentait une teneur en fécule de 36% sur matières sèches.

Ce gâteau incubé à 50°C à un pH de 5,0 pendant 12 heures, a été recyclé dans un milieu de culture principal dont la composition était:

- glucose　　　　 5%
- protéine de soja 1%
- C.S.L.　　　　　 1%.

L'activité enzymatique trouvée après 72 heures est égale à celle du milieu initial au moment de la première filtration.

Cette opération a pu être répétée une dizaine de fois sans affaiblissement notable de l'activité biologique recherchée et des performances en filtration.

Exemple 3
Culture de Penicillium Chrysogenum – Production de penicilline.

On cultive une souche particulière de Penicillium Chrysogenum sur un milieu contenant:

- 30 kg/m³ de Corn-Steep liquide, de la qualité décrite dans la demande de brevet français No 79 22106 déposée par la Société demanderesse le 4 septembre 1979,
- 20 kg/m³ d'hydrolysat enzymatique d'amidon, ayant un DE (dextrose-équivalent de 97) environ, commercialisé par la Société demanderesse, 10 kg/m³ de cet hydrolysat étant introduits en continu en cours de fermentation, suivant le processus décrit par V.F. DAVY et MARVIN JOHNSON dans «Application Microbiology», 1, pages 208 à 211 (1953), «Penicillin production in corn steep media with continuous carbohydrate addition»,
- un précurseur du type phényl-acétique.

Après une fermentation de 4 jours, le moût de culture est filtré sur un filtre rotatif du type DORR OLIVER garni de fécule de pomme de terre dont la qualité bactériologique est celle de la fécule utilisée en filtration brasserie ou en production de levure alimentaire.

La vitesse de filtration est de 300 à 400 l/m² /heure. Le mycélium ainsi séparé du milieu à 35°C et lavé sur le filtre ressemble à de la pulpe de papier grossière.

Le taux de matières sèches du gâteau de filtration est de 17% et la teneur de gâteau en fécule exprimée sur matières sèches de 27%.

La composition sur matières sèches du gâteau est:

| fécule | 27% |
|---|---|
| protéines | 28% |
| graisses | 7% |
| matières minérales | 14 |
| thiamine | 5,0 μg/gramme |
| riboflavine | 37,0 μg/gramme |
| acide pantothénique | 60,0 μg/gramme |
| acide nicotinique | 100,0 μg/gramme |
| acide folique | 5,0 μg/gramme |
| biotine | 3,0 μg/gramme |
| pyridoxine | 10,0 μg/gramme |

Le gâteau ainsi recueilli (82 kg/m³ de moût filtré) a été ajusté à un pH de 5,0 et porté sous légère agitation à 50°C, puis maintenu sous ces conditions d'autolyse pendant 12 heures.

L'autolysat a été amené à 100°C pour disperser complètement la fécule, puis la température a été ramenée à 60°C et on a ajouté 100 unités d'amyloglucosidase par litre d'autolysat.

Après 10 heures d'hydrolyse enzymatique, le taux de sucres réducteurs a été trouvé égal à 40 grammes/litre. Cet autolysat-hydrolysat a été recyclé en fermentation où il a remplacé 10 kg/m³ de C.S.L. et 10 kg/m³ d'hydrolysat d'amidon.

Cette opération a pu être répétée à plusieurs reprises sans baisse d'activité antibiotique et sans abaissement de la vitesse de filtration.

**Revendications**

1. Procédé microbiologique comportant une étape de filtration du moût qui permet de séparer, d'une part, un gâteau de filtration formé notamment des corps cellulaires du microorganisme mis en œuvre et de diverses particules et substances, notamment colloïdales, en suspension et, d'autre part, une phase liquide renfermant les principes actifs résultant du procédé microbiologique, ce procédé étant caractérisé par le fait que, consécutivement à la filtration du moût sur un agent de filtration de nature amylacée, le gâteau de filtration est recyclé dans le milieu de culture principal, constituant ainsi une partie des matières nutritives nécessaires à ce dernier, de préférence après avoir été soumis à un traitement de dégradation, notamment une autolyse.

2. Procédé selon la revendication 1, caractérisé par le fait que, pour augmenter la fermentescibilité du gâteau lors du traitement de dégradation, on peut le traiter à divers pH, diverses températures et ajouter tous types d'enzymes capables d'hydrolyser tout ou partie de ses constituants.

3. Procédé selon l'une des revendications 1 et 2, caractérisé par le fait que, pour le traitement de dégradation, le gâteau de filtration est recueilli tel quel ou délayé dans des cuves, amené à un pH compris entre 4,0 et 8,5 et à une température de 20 à 60°C.

4. Procédé selon l'une des revendications 1 à 3, caractérisé par le fait qu'au gâteau de filtration on ajoute des amylases capables d'hydrolyser le constituant amylacé du gâteau, notamment de l'α-amylase et de l'amyloglucosidase.

5. Application du procédé selon l'une des revendications 1 à 4 aux procédés de fermentation destinés à la préparation d'enzymes, de vitamines, d'antibiotiques, d'acides organiques ou d'acides aminés.

**Claims**

1. Microbiological process comprising a filtration step of the culture wort to separate, on the one hand, a filtration cake formed especially from the cell bodies of the microorganisms applied and from various particles and substances, especially of colloidal nature, suspended in the culture wort and, on the other hand, a liquid phase containing the active principles resulting from the microbiological process, the said process being characterized by the fact that, subsequently to the filtration of the culture wort on a filtration agent of amylaceous nature, the filter cake is recycled into the principal culture medium, thus constituting a portion of the nutrient matter necessary for the latter, preferably after having been subjected to a degradation treatment, especially an autolysis.

2. Process according to claim 1, characterized by the fact that, to increase the fermentability of the cake during the degradation treatment, one can treat the said cake at various values of pH, at various temperatures and one can add any type of enzyme capable of hydrolysing all or part of its constituents.

3. Process according to one of claims 1 and 2, characterized by the fact that, fo the degradation treatment, the filter cake is collected as such or diluted in tanks, its pH being brought to a value comprised between 4.0 and 8.5 and its temperature to a value of 20° to 60°C.

4. Process according to one of claims 1 to 3, characterized by the fact that amylases capable of hydrolysing the amylaceous constituent of the cake, especially α-amylase and amyloglucosidase are added to the filter cake.

5. Application of the process according to one of claims 1 to 4 to the fermentation processes intended for the preparation of enzymes, of vitamins, of antibiotics, of organic acids or of amino acids.

**Patentansprüche**

1. Mikrobiologisches Verfahren mit einer Filterstufe der Würze, welche die Trennung einerseits eines Filterkuchens bestehend insbesondere aus den Zellkörpern des in Anwendung gebrachten Mikroorganismus, sowie aus verschiedenen Teilchen und Substanzen, insbesondere kolloidaler Art, die aufgeschlämmt sind und andererseits einer flüssigen Phase, welche die von dem mikrobiologischen Verfahren herrührenden Wirkstoffe enthält, ermöglicht, wobei dieses Verfahren dadurch gekennzeichnet ist, dass anschliessend an die Filterung der Würze über ein Filtermittel stärkeartiger Natur, der Filterkuchen in das Hauptkulturmedium zurückgeführt wird und demzufolge einen Teil der Nährmaterialien darstellt, welcher letzterem notwendig sind, vorzugsweise nachdem er einer Abbaubehandlung, insbesondere einer Autolyse unterworfen wurde.

2. Verfahren nach Anspruch 1, dadurch gekennzeichnet, dass man zur Erhöhung der Vergärbarkeit des Kuchens während der Abbaubehandlung denselben bei verschiedenen pH-Werten und verschiedenen Temperaturen behandeln kann, und ihm alle möglichen Typen von Enzymen zufügen kann, welche imstande sind, all seine Bestandteile oder einen Teil derselben zu hydrolysieren.

3. Verfahren nach einem der Ansprüche 1 und 2, dadurch gekennzeichnet, dass der Filterkuchen für die Abbaubehandlung unverändert oder verdünnt in Behältern aufgenommen wird, auf einen pH-Wert zwischen 4,0 und 8,5 und auf eine Temperatur von 20 bis 60°C gebracht wird.

4. Verfahren nach einem der Ansprüche 1 bis 3, dadurch gekennzeichnet, dass man dem Filterkuchen zur Hydrolyse des stärkeartigen Bestandteils des Kuchens befähigte Amylasen, insbesondere α-Amylase und Amyloglucosidase zugibt.

5. Anwendung des Verfahrens nach einem der Ansprüche 1 bis 4, bei Verfahren, die zur Herstellung von Enzymen, Vitaminen, Antibiotika, organischen Säuren oder Aminosäuren bestimmt sind.